# EUROPEAN PATENT SPECIFICATION

(11) **EP 0 656 117 B1**
(45) Date of publication and mention of the grant of the patent: **02.02.2000**
(21) Application number: 94920582.7
(22) Date of filing: 23.06.1994
(51) Int. Cl.: G01N 33/68, G01N 33/74, C12N 15/12, C12N 15/18, C07K 14/655

(54) **A METHODOLOGY FOR SELECTING SUPERAGONISTS, ANTAGONISTS AND SUPERANTAGONISTS OF INTERLEUKIN 6**
METHODE ZUR AUSWAHL VON SUPERAGONISTEN, ANTAGONISTEN UND SUPERANTAGONISTEN VON INTERLEUKIN 6
METHODE DE SELECTION DE SUPER-AGONISTES, D'ANTAGONISTES ET DE SUPER-ANTAGONISTES D'INTERLEUKIN 6

(30) Priority: 23.06.1993 IT RM930409
(43) Date of publication of application: 07.06.1995
(73) Proprietor: ISTITUTO DI RICERCHE DI BIOLOGIA MOLECOLARE P. ANGELETTI S.P.A., 00040 Pomezia (IT)
(72) Inventor: SAVINO, Rocco, I-00040 Pomezia (IT); LAHM, Armin, I-00153 Roma (IT); CILIBERTO, Gennaro, I-00124 Casalpalocco (IT)
(74) Representative: Di Cerbo, Mario
(86) International application number: IT9400095
(87) International publication number: WO9500852

(56) References cited:
- EP-A- 0 411 946
- WO-A-90/04788
- WO-A-92/21029
- WO-A-94/09138
- US-A- 5 210 075
- EMBO JOURNAL, vol.13, no.6, 1994, HEIDELBERG FRG pages 1357 - 1367 'Generation of interleukin-6 receptor antagonists by molecular-modeling guided mutagenesis of residues important for gp 130 activation'

## Description

The present invention relates to a methodology for selecting superagonists, antagonists and superantagonists of Interelukin 6 whose receptor complex includes gp 130.

As is known, WO 92/21029 to Genentec Inc. teaches a method for determination of agonists or antagonists of growth hormones and ligands with a similar structural conformation. The potential agonists and antagonists are put into contact with a receptor for the hormone and this causes formation of a ternary complex consisting of a molecule of the potential agonist or antagonist and two molecules of such receptor for the hormone to be agonized or antagonized. Dimerization of receptors induced by a ligand molecule allows to conclude that the ligand has two different interaction sites (site 1 and site 2), on which it is possible to operate using mutagenesis to generate agonists or antagonists.

It has now been surprisingly found that the ligands in the group of cytokines similar to Interleukin 6 (IL-6), that is Oncostatin M (OSM), Leukemia Inhibitory Factor (LIF), Ciliary Neurotrophic Factor (CNTF), and Interleukin 11 (IL-11), induce the formation of a receptor complex of which the membrane molecule gp 130 is a part. In this receptor complex the specific receptor for each of these cytokins and the membrane molecule gp 130 are always present as common elements. It is thus possible to formulate the hypothesis that site 1 and site 2 bind to two different molecules in this class of hormones: site 1 to the specific receptor and site 2 to gp 130.

Identification of the two sites is made possible, as will be seen more clearly from the following, by construction of a three-dimensional model of the receptor complex based on the functional similarity between sequences of the human growth hormone receptor (hGH) and sequences of the receptors for the hormones in question. Isolation of variants that, with respect to the wild type hormone, have a greater affinity for the specific receptor (superagonists or superantagonists) is obtained by construction of filamentous phage libraries, for example M13, carrying the hormone, both in the wild type and mutant version.

The difference between the three-dimensional model, for example of IL-6, adopted here and the one adopted in WO92/21029 leads to hypothesize different residues in helix A and C as constituents of site 2.

Modelling of the human interleukin 6 molecule is performed as follows. Knowing, from data available in scientific literature, that human interleukin 6 belongs to a class of cytokines that have four helices forming the core of their three-dimensional structure, the amino acidic sequence of human interleukin 6 was analyzed to identify the four regions in which there was the highest probability of a helix formation. In a following stage, these four helix regions of the interleukin 6 molecule were modelled in a computerized interactive graphic unit. To start, it was assumed that the orientation of the four helices might be similar to that seen in hormones such as the growth hormone or the macrophage granulocyte colony stimulation factor. To optimize packaging of the hydrophobic amino acids in the space between the four helices, adjustments to the relative positions of the helices were made. Subsequently, the loops connecting the four helices were modelled.

This three-dimensional model of interleukin 6 has enabled the identification of the two sites of interaction between human interleukin 6 and its two receptors: the low affinity receptor gp 80 (site 1) and the high affinity receptor for gp 130 signal transduction (site 2). The following procedure was used to identify the two sites. From comparison of sequences it is known that all the members of one family of hematopoietic receptors are related to each other by the fact that they share a domain, known as the cytokine recognition domain. This similarity of sequences also indicates a high probability of structural similarity in corresponding parts of the various receptors, including the two interleukin 6 receptors, gp 80 and gp 130. The observation that the cytokines that bind to these receptors all have (or can be predicted to have) a similar structure, that is a four helix matrix, strongly supports the hypothesis that the interaction between these cytokines and their receptors, by means of the cytokin recognition domain, must be very similar in biologically active complexes.

Considering that the three-dimensional structure of one of these compounds (the complex made by growth hormone and the extra-cellular domain of the dimeric receptor for the growth hormone) has been determined by means of X-ray crystallography, our model of human interleukin 6 allows us to identify the potential sites of interaction between interleukin 6 and its two receptors gp 80 (site 1) and gp 130 (site 2). This by comparison with the complex involving the growth hormone and assuming that the functionally important amino acids are located in similar positions on the surface of the two hormones.

The need to provide a methodology the production of agonists, antagonists and superantagonists for hormones of the immune system whose receptor complex includes gp 130, will be explained with reference to the case of interleukin-6.

As is known, interleukin 6 is a polypeptide of 184 amino acids which, as described, belongs to the class of helical cytokines. Interleukin 6 is a multi-functional cytokine produced by various types of cell. It acts as a differentiation and growth factor on cells of various types, such as for example cells in the immune system, hepatocytes, kidney cells, hemopoietic staminal cells, keratinocytes and neurones.

Production of superagonists of interleukin 6 would allow the use of lower therapeutic doses than those required with wild interleukin 6 in the treatment of numerous serious diseases. In fact, interleukin 6 has important and promising applications in the treatment of breast cancer, leukemia, and infectious diseases or diseases connected with disorders of the cells producing bone marrow.

On the other hand the production of superantagonists or superantagonists of human interleukin 6 would allow inhibition of interleukin 6 in numerous diseases characterized by its excessive production, such as chronic autoimmune diseases, myeloma/plasmacytoma, post-menopausal osteoporosis and cancer cachexy.

The methodology for the selection of superagonists, antagonists or superantagonists of a Interleukin 6 using the membrane molecule gp 130 to activate the mechanisms regulating cell physiology, according to the present invention, comprises the following operations:
- comparing the amino acid sequences of the growth hormone with the sequences of said interleukin 6;
- comparing the amino acid sequences of the growth hormone receptor with those of the two receptors of the interleukin 6, that is with interleukin 6-specific receptor and gp 130;
- on the basis of the above comparisons, formulating a three-dimensional model of the receptor complex based on the functional similarity between sequences of the growth hormone receptor and the two interleukin 6 receptors in question; and
- identifying the residues of the wild type interleukin 6 that are a part of the site of interaction with the specific receptor and of the site of interaction with gp 130, respectively;
- introduction of mutations into the interaction sites of interleukin 6; and
- evaluation of the biological activity of the mutants.

For selection of superagonists of interleukin 6, the methodology according to the present invention further comprises the following additional operations:
- production of a series of phage libraries containing mutations of the following wild type residues of interleukin 6, present in the form of fusion product with filamentous phage proteins
   Glu 42, Glu 51, Ser 52, Ser 53, Lys 54, Glu 55, Asn 63, Lys 66, Met 67, Ala 68, Glu 69, Lys 70, Asp 71, Phe 170, Gln 175, Ser 176, Ser 177, Leu 181, Gln 183;
- generation of a phage library, each phage having a mutant interleukin 6 sequence;
- selection, from the mixed population of phages expressing interleukin 6 mutants, of that or those with an affinity for the specific receptor greater than that of wild type interleukin; and
- identification of the best amino acid sequence or sequences binding the receptor by sequencing of the DNA extracted from the selected phage particles.

In this case, a series of phage libraries can be produced containing mutations of said wild residues of interleukin 6 present as a product of fusion with the protein pIII of M13.

The methodology for selecting antagonists of interleukin 6 according to the present invention comprises - along with the operations indicated above - the following operations:
- mutation of the residues identified in claim 1, to form part of the site of interaction with gp 130 (Arg 30, Tyr 31, Gly 33, Ser 37, Ala 38, Ser 118, Lys 120, Val 121, Gln 124, Phe 125, Gln 127, Lys 128 and Lys 129), using conventional molecular biology techniques;
- evaluation of biological activity and affinity for the specific interleukin 6 receptor of the mutants produced as above, in order to identify variants of interleukin 6 whose affinity to the specific receptor is intact and that show reduction or loss of the biological activity; and
- evaluation of the above variants of interleukin 6 as antagonists for the biological activity of wild interleukin 6.

In case of selection of super-antagonists of interleukin 6 by combination of the variations of amino acid sequences responsible for antagonist activity, indicated above, with amino-acid variations responsible for an increased affinity of the specific receptor for interleukin 6.

In the methodology for selecting superantagonists of interleukin 6, the mutagenesis of the residues identified as above can be performed using a molecular biology technique chosen from the group comprising Polymerase Chain Reaction, Primer Extension, Oligonucleotide Directed Mutagenesis, and combinations thereof.

Up to this point a general description of the subject of the present invention has been given. With the aid of the following examples a detailed description of specific embodiments of the invention will now be given, with the purpose of giving a better understanding of the objects, characteristics, advantages and methods of application thereof.

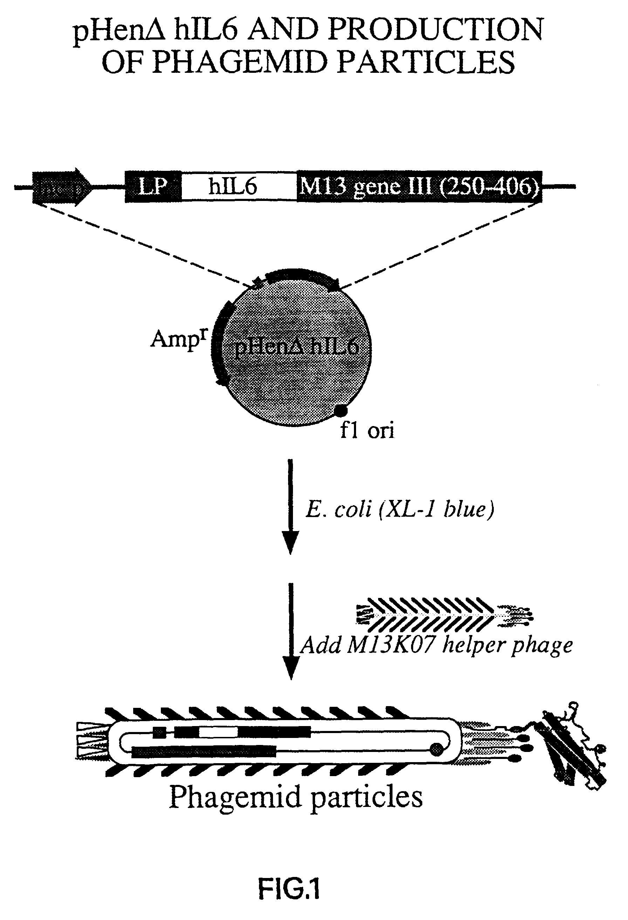

Figure 1 shows the construct pHen△hIL-6 and the production of phasmidic particles (see example 1, "Vector Construction"), used for selection of agonists of hIL-6.
Figure 2 shows the antagonistic activity of mutant IL-6 Tyr31Asp/Gly35Phe/Ser118Arg/Val121Asp with the increase of its concentration.

### DEPOSITS

E.Coli K12 bacteria - transformed using the plasmid pHen△hIL-6 containing, from the site for recognition of the restriction enzyme SalI to that for the restriction enzyme NotI, a nuleotidic sequence coding for the amino acid sequence of wild type human interleukin-6 - have been deposited on 10/6/1993 with The National Collection of Industrial and Marine Bacteria Ltd. (NCIMB), Aberdeen, Scotland, UK, with access number NCIMB 40563.

### Example 1

### Application of the methodology according to the present invention for the selection of agonists of interleukin-6

### 1) VECTOR CONSTRUCTION

The strategy consists in construction of a hybrid gene containing all the region coding for hIL-6 followed by the last 157 amino acids of protein pIII of the phage M13 and preceded by the sequence Pel B, which vectors the synthesized protein to the periplasmic space.

Expression of the hybrid gene is driven by the promotor lacZ. The construction is made in the context of vector pHen△e, and takes the name of pHen△hIL-6 (see the only figure enclosed). This plasmid also contains a phage replication origin. If a bacterial cell containing this plasmid is infected by a bacteriophage known as a "helper", such as M13K07, single filament copies will be produced by the plasmid, and will be coated with the phage proteins, just like a true phage genome. These phage particles containing the plasmid are known as phasmids. Along with the normal pIII molecules, they also contain hIL6-pIII fusion molecules. A hIL-6 molecule and the gene coding its amino acid sequence are thus contained within the same unit. In the case of mutant molecules, it will be possible to determine the amino acid sequence of the molecules exposed on the surfaces of the phages obtainied from selection processes, simply by sequencing the phasmid DNA.

A further characteristic of the construction pHen hIL-6 is the presence of a translation codon stop between the IL-6 gene and the pIII gene. Production of the hybrid protein is carried out in bacterial strains capable of suppressing this Stop codon. Vice versa the use of non-suppressor strains allows production of hIL-6 alone, directly in the periplasmic space.

The following experiments demonstrate the ability to use shrIL-6R to purify, from among the vast range of mutant interleukin-6 exposed on the phage using pHen hIL-6, those having the greatest affinity with said receptor, by means of amplification selection cycles.

### 2) SYSTEM CHARACTERIZATION EXPERIMENTS

### a) ELISA Test

The wells in ELISA plates were coated with hIL-6 phasmids or with M13K07 and made to react with shrIL-6R (soluble Human Recombinant IL-6R). After repeated washing the presence of the receptor was indicated using a specific monoclonal antibody conjugated with alkaline phosphatase. The signal obtained is greater in the case of hIL-6 phasmids and increases as the amount of receptor used increases.

### b) Enrichment of the hIL-6 phasmid with phasmid-K07 mixtures using shrIL-6R

hIL-6 phasmid particles were mixed with particles of M13K07 at a ratio of 1:100. This mixture was incubated with polystyrene balls coated with shrIL-6R. After repeated washing at a neutral pH the balls underwent stringent washing at pH 4.2, followed by elution at pH 3.6. The proportion of hIL-6 phasmids:M13K07 in the resulting eluant was then determined. The proportion is found to be 1:10, with a consequent enrichment by ten times of the hIL-6 phasmids with respect to M13K07.

### c) Selection from mixtures of mutant interleukin-6 of those having the highest affinity for the receptor gp 80

Phasmid particles were produced having on their surface mutant molecules of hIL-6 with a higher (176 Arg) or lower (179 Ala) affinity with the receptor, compared with the natural version. These particles were mixed in a ratio of 1:1. The mixture was incubated with the receptor in a solid phase (as described in point b). In the eluate at pH 3.6, the ratio between the two types of particle was found to be 15:1 in favor of 176 Arg.

### d) Determination of the relative affinity of M13K07, hIL-6 phasmid, 176 Arg phasmid and 179 Ala phasmid particles for the receptor gp 80

Equal amounts of particles of the various types listed above were incubated separately with the balls coated with receptor. After the usual washing sequence the number of particles recovered in the eluant at 3.6 was determined for each type of phage. Considering the value of particles recovered for the phasmid hIL-6 as 1, a value of 3 is obtained for 176 Arg, 0.2 for 179 Ala and 0.18 for M13K07. These values reflect the relative affinities of the molecules of IL-6 non exposed on the phage. It is, in fact, known that 176 Arg binds the receptor with an affinity three times greater than that of the natural molecule, whereas in the case of 179 Ala the affinity for the receptor is reduced almost to zero (in fact a phasmid with this mutant, as regards binding to the receptor, behaves like M13K07).

This group of experiments has shown that it is possible, using the method according to the present invention, to select from mixtures of mutants exposed on phage those having the greatest affinity for the receptor.

### Example 2

### Generation and selection of antagonists of interleukin 6 using the methodology according to the present invention

The plasmid Phen△hIL-6 was used as a template for all the mutagenesis reactions. This plasmid is a derivative of the plasmid pHEN1 and contains the area coding for human IL-6 (SEQ ID NO:1) upstream the area coding for the carboxy-terminal portion (from codon 250 to the COOH end) of the protein pIII of the bacteriophage M13; the two coding areas are in reading frame and are separated by an Amber UAG Stop codon, which can be suppressed in bacterial strains that have the gene of the suppressor SupE integrated in their genome. The area coding for human IL-6 is also in reading frame, below the peptide PelB, a signal sequence for secretion, and the whole gene is under the control of the promotor LacZ. Finally, a single site for the restriction enzyme SacI has been introduced in the nucleotide sequence coding for the amino acids 20-21-22 of hIL-6 without changing their identity and, equally, a single site for the restriction enzyme BfrI has been introduced in the area coding for the amino acids 38-39-40 of hIL-6, likewise without changing them.

A PCR strategy (Polymerase Chain Reaction) was used to generate mutations within the codons selected in the area coding for human interleukin 6. The primer downstream is H9, a 32 nucleotide primer, corresponding to positions 426-457 (antisense filament) of hIL-6 cDNA (taking the first nucleotide of the first codon of the mature polypeptide to be 1). The primer hybridization site is downstream the recognition site for the enzyme XbaI, naturally present in the cDNA. The mutagenetic primer upstream is IL-6 31D/35 PCR, a 70 nucleotides primer, whose sequence is SEQ ID NO:2.

The primer IL-6 31D/35 PCR extends from position 55 to position 124 (sense filament) of the hIL-6 cDNA and introduces degenerations into the codons coding for the amino acid 31 (wild type tyrosine) and 35 (wild type glycine). A DNA fragment of 403 pairs of bases is amplified using PCR according to standard PCR amplification protocols. Amplification is performed in 35 cycles. Each cycle consists of incubation for 2 minutes at 94°C for denaturation of the template, 2 minutes at 50°C for hybridization of the oligonucleotide and 3 minutes at 72°C for extension of the chain. The amplified fragment is digested with SacI and XbaI and purified using 2% agarose gel. The fragment generated by PCR and digested by the two enzymes is ligated into the vector pHen hIL-6 digested with the same two enzymes, purified on 0.8% agarose gel, to replace the wild type sequence.

The following Table 1 shows the biological activity, in human hepatoma cells, and binding to the receptor gp 80 for wild type interleukin-6 and mutations thereof, the mutations being indicated with an asterix.

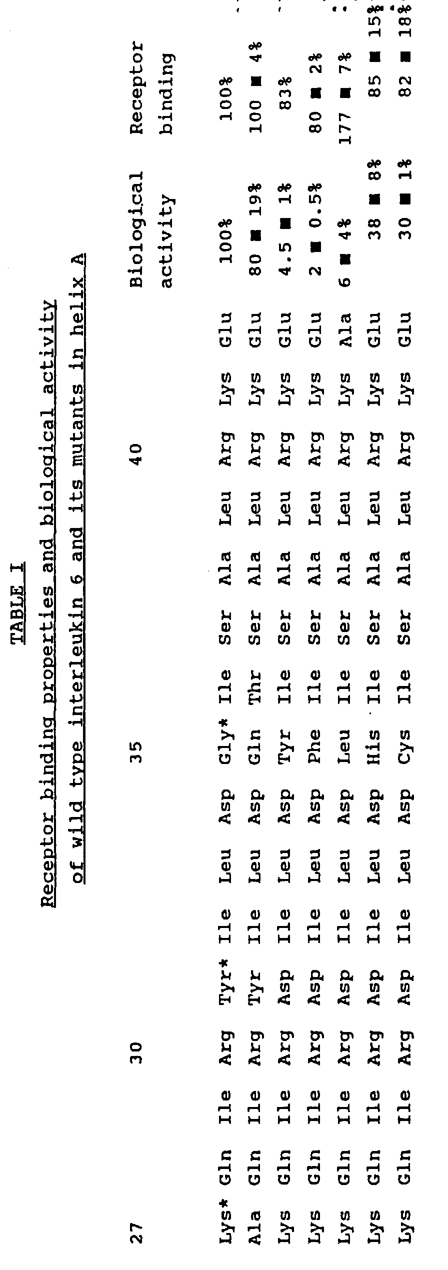

As can be seen from the table, the mutants (Tyr31Asp, Gly35Tyr), (Tyr31Asp, Gly35Phe) and (Tyr31Asp, Gly35Leu, Glu42Ala) show lower biological activity when compared with wild type interleukin 6. In all three cases the residual activity is 2-5% that of wild type interleukin-6. The three mutants maintain their full ability to bind to the interleukin 6 receptor gp 80. In conclusion, the three mutants have a decreasing signal transduction activity in hepatoma cells. In other words, they are antagonists of wild interleukin 6. In particular, the mutant (Tyr31Asp, Gly35Phe) is a particularly effective antagonist, as it is capable of reducing the activity of wild type interleukin 6 when used at 50 fold molar excess in tests on human hepatoma cells. Example 3 Generation and selection of further antagonists of interleukin 6 using the methodology according to the present invention The plasmid pHENΔhIL-6, described in the preceding example, was used as a template for all the mutagen reactions. A PCR strategy (Polymerase Chain Reaction) was used to generate mutations within the codons selected for the area coding for human interleukin 6. The primer above is HP/1, a primer with 29 nucleotides, corresponding to positions 1-19 (sense filament) of hIL-6 cDNA (taking the first nucleotide of the first codon of the mature polypeptide to be 1). The primer hybridization site is uspstream to the site recognizing the enzyme SacI, artificially introduced in the cDNA without changing the sequence coded by the latter, as described in example 2. The mutagenetic primer below is IL-6 118RCLF/121VD, a primer with 72 nucleotides, whose sequence is SEQ ID NO 3. The primer IL-6 118RCLF/121VD extends from position 334 to position 405 (antisense filament) of the cDNA of hIL- 6 and introduces degenerations into the codons coding for the amino acid 118 (wild type serine) and 121 (wild type valine). A DNA fragment of 415 pairs of bases is amplified using PCR according to standard PCR amplification protocols. Amplification is performed in 35 cycles. Each cycle consists of incubation for 2 minutes at 94°C for denaturation of the template, 2 minutes at 50°C for hybridization of the oligonucleotide and 3 minutes at 72°C for extension of the chain. The amplified fragment is digested with SacI and XbaI and purified using 2% agarose gel. The fragment generated by PCR and digested by the two enzymes is ligated into the vector pHen△hIL-6 digested with the same two enzymes, purified on 0.8% agarose gel, to replace the wild type sequence.

The following Table 2 shows the biological activity, in human hepatoma cells, and binding to the receptor for wild interleukin-6 and versions thereof with mutations in the residues indicated.

**TABLE 2**

| Receptor binding properties and biological activity of wild-type interleukin 6 and helix C mutations thereof | | | |
|---|---|---|---|
| 118 Ser | 121 Val | Biological activity 100% | Receptor binding 100% |
| Arg | Val | 66% | 81% |
| Leu | Asp | 36■4% | 92% |
| Arg | Asp | 3.5■0.5% | 66■5% |
| Ser | Asp | 58■23% | 78■2% |

It can be seen that the mutant IL-6 Ser118Arg/Val121Asp has characteristics very similar to those of the mutant IL-6 Tyr31Asp/Gly35Phe, described in Table 1, that is to say it binds normally to the type I receptor of interleukin 6, but cytokin biological activity is reduced approximately thirty-fold.

### Example 4

### Generation and selection of more powerful antagonists of interleukin 6 using the methodology according to the present invention

In this case, the plasmid pHEN△hIL-6 Tyr31Asp/Gly35Phe, obtained as described in Example 2, was used as a template for all the mutagenic reactions.

A PCR strategy (Polymerase Chain Reaction) was used to generate mutations within the codons selected for the area coding for human interleukin 6. The primer above is HP/1, described in the previous example. The mutagenetic primer below is IL-6 118RCLF/121VD, also described in the previous example. A DNA fragment of 415 pairs of bases is amplified using PCR according to standard PCR amplification protocols. Amplification is performed in 35 cycles. Each cycle consists of incubation for 2 minutes at 94°C for denaturation of the template, 2 minutes at 50°C for hybridization of the oligonucleotide and 3 minutes at 72°C for extension of the chain. The amplified fragment is digested with SacI and XbaI and purified using 2% agarose gel. The fragment generated by PCR and digested by the two enzymes is ligated into the vector pHen hIL-6 digested with the same two enzymes, purified on 0.8% agarose gel, to replace the wild type sequence.

The following Table 3 shows the biological activity, in human hepatoma cells, and binding to the receptor for wild interleukin-6 and versions thereof with mutations in the residues indicated.

**TABLE 3**

| Receptor binding properties and biological activity of wild interleukin 6 and helix A and C mutants thereof | | | | | |
|---|---|---|---|---|---|
| Helix A | | Helix C | | | |
| 31 | 35 | 118 | 121 | Biological activity | Receptor binding |
| Tyr | Gly | Ser | Val | 100% | 100% |
| Asp | Phe | Leu | Val | 1.4% | N.D. |
| Asp | Phe | Arg | Val | 5.4■1.1% | 66■2% |
| Asp | Phe | Leu | Asp | 0% | 63■4% |
| Asp | Phe | Arg | Asp | 0% | 97■15% |
| Asp | Phe | Phe | Asp | 0% | 76■26% |

| | | | | | |
|---|---|---|---|---|---|
| N.D. = Not determined | | | | | |

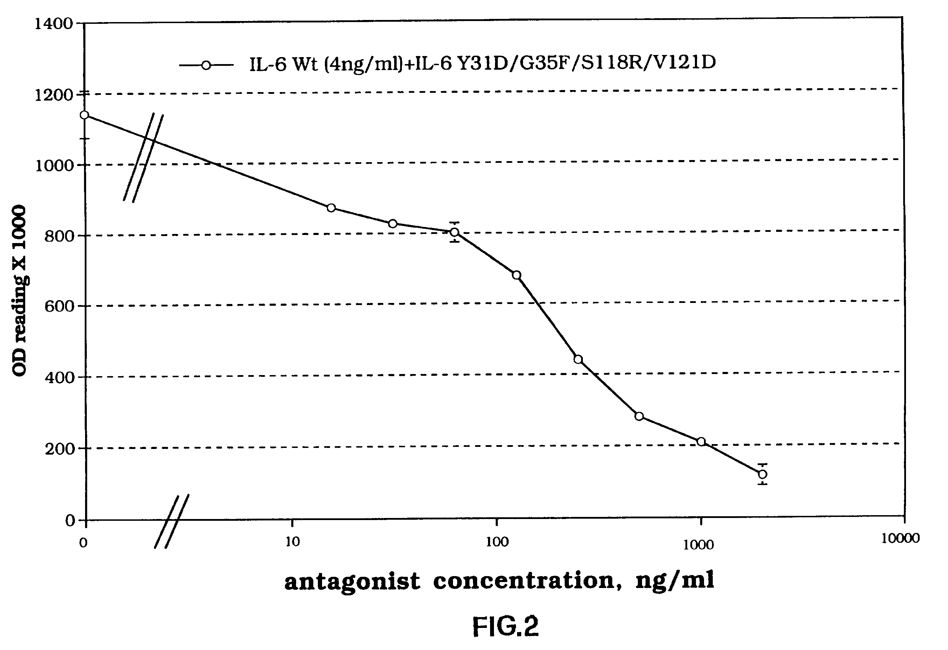

Three of the variants containing mutations both on helix A and on helix C show no sign of biological activity in human hepatoma cells, whereas they maintain their ability to bind the receptor gp80. Among these three proteins, the mutant IL-6 Tyr31Asp/Gly35Phe/Ser118Arg/Val121Asp was chosen for competition experiments with wild type interleukin 6 biological activity in human hepatoma cells. The cells were stimulated with wild interleukin 6 at 4 nanograms per milliliter (ng/ml) of culture medium, in the presence of increasing concentrations of mutant. As illustrated in figure 2 (in which biological activity of wild type interleukin 6 is expressed in arbitrary units), increasing concentrations of the mutant manage to fully antagonize the effects of wild interleukin 6 on human hepatoma cells.

Table 4 shows the levels of inhibition (in percentage) of the biological activity of wild interleukin 6 according to the concentration of antagonist (expressed in nanograms per milliliter, molar excess compared with wild type interleukin 6 and nanomoles per liter, respectively).

**TABLE 4**

| Inhibition of the biological activity of wild interleukin 6 according to the concentration of antagonist | | | |
|---|---|---|---|
| IL-6 g/ml | Tyr31Asp/Gly35Phe/Ser118Arg/Val121Asp | | Inhibition of wild IL-6 (%) |
| | molar excess | concentration (nM) | |
| 16 | 4 X | 0.7 nM | 23% |
| 60 | 15 X | 2.7 nM | 28% |
| 164 | 41 X | 7.5 nM | 50% |
| 500 | 125 X | 22.7 nM | 75% |
| 1000 | 250 X | 45.5 nM | 82% |
| 2000 | 500 X | 90.0 nM | 90% |
| 4000 | 1000 X | 181.8 nM | 96% |

### Example 5

### The interleukin 6 antagonist, generated and selected using the methodology described in the present invention, inhibit interleukin 6-dependent growth of human myeloma cells.

In the previuos example it was shown that one of the mutants, namely IL-6 Tyr31Asp/Gly35Phe/Ser118Arg/Val121Asp was able to inhibit interleukin 6 biological activity (stimulation of transcription by an interleukin 6 inducible promoter) on human hepatoma cells. In the introductory part of this patent application it is stated that development of interleukin 6 antagonists or superantagonists would have practical application because they could be used to inhibit interleukin 6 activity in diseases characterized by interleukin 6 overproduction, like various forms of multiple myeloma/plasmacytoma. We provide here a further example by showing that the three mutants
IL-6 Tyr31Asp/Gly35Phe/Ser118Arg/Val121Asp (DFRD),
IL-6 Tyr31Asp/Gly35Phe/Ser118Phe/Val121Asp (DFFD),
IL-6 Tyr31Asp/Gly35Phe/Ser118Leu/Val121Asp (DFLD)
fully inhibit the interleukin 6-dependent growth of a human myeloma cell line, called XG-1, derived from freshly isolated myeloma cells from a patient with terminal disease. The XG-1 myeloma cell line growth is strictly dependent on exogenously added interleukin 6, similarly to what has been shown for fresh myeloma cells, therefore this cell line can be considered an excellent in vitro model of the multiple myeloma disease (Jourdan, M., Zhang, X-G., Portier, M., Boiron, J.-M., Bataille, R. and Klein, B.(1991) J. Immunol. 147, 4402-4407). To test mutants antagonism on wild type interleukin 6, XG-1 myeloma cells were coltured in 96-well microtiter plates at 6000 cell/microwell with wild type interleukin 6 at 0.1 nanograms per milliliter (ng/ml) of colture medium, in the presence of increasing concentrations of each one of the three mutants. After 7 days of colture, cell numbers were evaluated by colorimetric determination of hexosaminidase levels (Landegren, U. (1984) J. Immunol. Methods 67, 379-388). The following Table 5 shows the inhibition of wild type interleukin 6 activity as a function of the three antagonist concentrations (expressed in nanograms of mutant per milliliter of colture medium).

**TABLE 5**

| Inhibition of wild type interleukin 6 activity (stimulation of growth of XG-1 human myeloma cells) as a function of the three antagonists concentration. | | | |
|---|---|---|---|
| Antagonist | Inhibition of wild type interleukin 6 produced by concentration | | |
| | DFRD | DFFD | DFLD |
| 3.3 ng/ml | 6% | 0% | 0% |
| 10 ng/ml | 12% | 7% | 3% |
| 30 ng/ml | 25% | 18% | 10% |
| 90 ng/ml | 42% | 35% | 31% |
| 270 ng/ml | 64% | 59% | 53% |
| 810 ng/ml | 84% | 84% | 71% |
| 2430 ng/ml | 90% | 90% | 76% |
| 7290 ng/ml | 93% | 95% | 81% |

As can be seen from the table, all mutants are particularly effective antagonists of interleukin 6 biological activity on human myeloma cells.

### Example 6

### Application of the methodology according to the present invention for the selection of novel superagonists of interleukin 6

A phage library (containing mutations of residues Gln 175, Ser 177, Leu 181 and Leu 183 of wild type interleukin 6, present in the form of fusion product with filamentous phage protein) was constructed using the Primer Extension molecular biology technique The mutagenetic oligonucleotide is IL-6 QSLQ (AS), a 62 nucleotides oligo, whose sequence is SEQ ID NO:4. Primer IL-6 QSLQ (AS) extends from position 507 to the stop codon of the interleukin 6 cDNA (antisense strand), it introduces degenerations into codons coding for the amino acids 175 (wild type Gln), 177 (wild type Ser), 181 (wild type Leu) and 183 (wild type Gln) and it also introduces a NotI restriction site downstream of the interleukin 6 stop codon. Oligonucleotide IL-6 QSLQ pr. Bam, whose sequence is SEQ ID NO:5, was used as primer for the Primer Extension reaction. Oligonucleotide IL-6 QSLQ pr. Bam extends from position 503 to position 522 (sense strand) of the interleukin 6 cDNA and it contains a BamHI recognition site within the 5' nine nucleotides. The two oligonucleotides are complementary to each other on a region corresponding to position 507 through position 522 of the interleukin 6 cDNA. The two oligonucleotides were annealed in vitro, and the annealed oligonucleotides were used as substrate for a Primer Extension reaction, performed using the Klenow enzyme. The double-stranded DNA fragment thus obtained was then digested with BamHI (compatible with BglII) and with NotI and ligated into the plasmid phen△hIL-6, digested with BglII (compatible with BamHI) and with NotI, in order to replace the wild type sequence with the mutated ones. The ligation product was inserted in bacteria, yielding roughly one million independent transformants ("trasformant" is the definition given to a bacterium which has incorporated a recombinant plasmid). The transformed bacteria were infected by with the M13K07 helper bacteriophage to generate the phage library (a library of phasmids) as described in the example 1.

The library underwent selection by incubation with polystirene balls coated with shrIL-6R, as described in the example 1. The phage population eluted at pH 3.6 was then amplified in bacteria. After five cycles of selection-amplification, randomly selected phages were seqenced over the mutagenized region, the corresponding mutant interleukin 6 proteins were produced in the periplasmic space of the appropiate bacterial strain (as described in the example 1) and tested for both receptor binding and biological activity on human hepatoma cells. Table 6 below shows that, by using using the methodology to the present invention, it is possible to select superagonists of interleukin 6, mutant molecules which have increased both receptor binding and biological activity on human hepatoma cells.

**TABLE 6**

| Receptor binding properties and biological activities of wild type intyerleukin 6 and of its mutants in the helix D | | | | | | |
|---|---|---|---|---|---|---|
| Position | 175 | 177 | 181 | 183 | Receptor binding (%) | Biological activity (%) |
| wild type | Gln | Ser | Leu | Gln | 100% | 100% |
| phage 5-4 | Gln | Ser | Leu | Tyr | 240% | 130% |
| phage 5-8 | Gln | Ser | Leu | Ala | 240% | 120% |
| phage 5-2 | Ile | Ser | Leu | Ala | 260% | 150% |
| phage 4-8 | Gln | Ser | Ile | Asn | 100% | N. D. |
| phage 5-3 | Ile | Ser | Val | His | 80% | N. D. |

| | | | | | | |
|---|---|---|---|---|---|---|
| N. D.: not determined | | | | | | |

The mutations selected by the methodology according to the present invention can be used as starting point for the development of more potent interleukin 6 superagonists. This is shown in this example, in which the mutation identified in the phage 5-2 are combined with the mutation Ser176Arg, which by prior art is known to increase both receptor binding and biological activity (see International Publication WO 94/11402 of a PCT application of the present Applicant, filed 2.11.93 with Italian priority of 6.11.92). The three mutations were grouped on the same cDNA by mean of Oligonucleotide Directed Mutagenesis. Oligonucleotide 175I/176R/183A (S), whose sequence is SEQ ID NO:6 is 73 nucleotides long which extends from position 499 to the stop codon (sense strand) of the interleukin 6 cDNA present in phen hIL-6 and which has a recognition site for the enzyme NotI downstream of the stop codon. Oligonucleotide 175I/176R/183A (AS), whose sequence is SEQ ID NO:7, is 73 nucleotides long which extends from position 499 to the stop codon (antisense strand) of the interleukin 6 cDNA present in phen△hIL-6 and which has a recognition site for the enzyme NotI upstream of the stop codon. The two oligonucleotide are complementary to each other and they both encode the amino acid Isoleucine in position 175, the amino amino acid Serine in position 176 and the amino acid Alanine in position 183. The two oligonucleotides were annealed in vitro, the double-stranded DNA fragment thus obtained was digested with the restriction enzymes BglII and Not I and ligated into the vector phen△hlL-6 digested with the same two enzymes, in order to replace the wild type sequence with the mutated one. The corresponding interleukin 6 variant carrying the three desired mutations was produced in the periplasmic space of the appropiate bacterial strain (as described in the example 1) and tested for both receptor binding and biological activity on human hepatoma cells. The following Table 7 shows the receptor binding properties and the biological activity on human hepatoma cells of both the new triple mutant and of the parental double mutant.

**TABLE 7**

| Receptor binding properties and biological activities of wild type intyerleukin 6 and of both double and triple mutants in helix D | | | | | |
|---|---|---|---|---|---|
| Position | 175 | 176 | 183 | Receptor binding (%) | Biological activity (%) |
| wild type | Gln | Ser | Gln | 100% | 100% |
| phage 5-2 | Ile | Ser | Ala | 260% | 150% |
| triple mutant | Ile | Arg | Ala | 450% | 260% |

As can be seen from the table, the mutant carrying the substitutions Gln175Ile/Ser176Arg/Gln183Ala is a much more effective interleukin 6 superagonist than the parental mutant carrying only the two substitutions Gln175Ile/Gln183Ala.

### Example 7

### Application of the methodology according to the present invention for the selection of superantagonists of interleukin 6

The three mutations Gln175Ile/Ser176Arg/Gln183Ala, identified in the example 6, which strongly increases the receptor binding capacity, were combined with the four mutations Tyr31Asp/Gly35Phe/Ser118Arg/Val121Asp, described in examples 4 and 5, which show the strongest antagonistic behaviour, by mean of a PCR strategy. The corresponding mutant protein,called SAnt 1 and containing all the seven mutations, was tested both for receptor binding and for antagonistic behaviour on human hepatoma and myeloma cells, the following table 8 shows the receptor binding properties of both SAnt 1 and DFRD (the mutant from which SAnt 1 was derived), together with the amounts (in nanograms of mutant per milliliter of colture medium) of mutant necessary to inhibit 50% of interleukin 6 biological activity (hepatoma cells were stimulated with 4 nanograms of wild type interleukin 6 per milliliter of colture medium, while myeloma cells were stimulated with 0.1 nanograms of interleukin 6 per milliliter of colture medium, due to the higher sensitivity of the latter cells to wild type interleukin 6).

**TABLE 8**

| Inhibition of wild type interleukin 6 activity on both human hepatoma and myeloma cells as a function of the antagonists receptor binding capacity. | | | | |
|---|---|---|---|---|
| Antagonist | Receptor binding (% of wt) | 50% inhibition of interleukin 6 hepatoma cells | | activity on myeloma cells |
| | | Hep3B | HepG2 | XG-1 |
| DFRD | 97% | 164 ng/ml | 132 ng/ml | 190 ng/ml |
| SAnt1 | 406% | 19 ng/ml | 32 ng/ml | 22 ng/ml |

As can be seen from the table, the introduction of the three mutations described in the example 6 (Gln175Ile/Ser176Arg/Gln183Ala) has at once increased the receptor binding capacity of the parental mutant DFRD and strongly decreased the amount of antagonist needed to inhibit 50% of interleukin 6 biological activity on all cell lines tested, therefore generating a very effective interleukin 6 superantagonist.

SEQUENCE LISTING
GENERAL INFORMATION
(i) APPLICANT: ISTITUTO DI RICERCHE DI BIOLOGIA
MOLECOLARE P. ANGELETTI S.p.A.
(ii) TITLE OF INVENTION: METHOD FOR SELECTING
SUPERAGONISTS, ANTAGONISTS AND SUPERANTAGONISTS FOR
HORMONES HAVING GP 130 AS PART OF THEIR RECEPTOR
COMPLEX
(iii) NUMBER OF SEQUENCES: 7
(iv) CORRESPONDENCE ADDRESS:
(A) ADDRESSEE: Società Italiana Brevetti
(B) STREET: Piazza di Pietra, 39
(C) CITY: Rome
(D) COUNTRY: Italy
(E) POSTAL CODE: I-00186
(viii) ATTORNEY INFORMATION
(A) NAME: DI CERBO, Mario (Dr.)
(C) REFERENCE: RM/090075/MDC
(ix) TELECOMMUNICATION INFORMATION
(A) TELEPHONE: 06/6785941
(B) TELEFAX: 06/6794692
(C) TELEX: 612287 ROPAT
(1) INFORMATION FOR SEQ ID NO: 1
(i) SEQUENCE CHARACTERISTICS
(A) LENGTH: 555 base pairs
(B) TYPE: nucleotide
(C) STRANDEDNESS: double
(D) TOPOLOGY: linear
(ii) MOLECULE TYPE: cDNA
(iii) HYPOTHETICAL: no
(iv) ANTISENSE: no
(v) FRAGMENT TYPE: internal
(vii) IMMEDIATE SOURCE
(A) SYNTHESIS: production in bacteria
(ix) FURTHER CHARACTERISTICS
(A) NAME: IL-6 cDNA
(C) IDENTIFICATION METHOD: polyacrylamide gel

(xi) SEQUENCE DESCRIPTION: SEQ ID NO: 1
CCA GTA CCC CCA GGA GAA GAT TCC AAA GAT GTA GCC GCC CCA CAC AGA 48
Pro Val Pro Pro Gly Glu Asp Ser Lys Asp Val Ala Ala Pro His Arg
1               5                   10                  15
CAG CCA CTC ACG AGC TCA GAA CGA ATT GAC AAA CAA ATT CGG TAC ATC 96
Gln Pro Leu Thr Ser Ser Glu Arg Ile Asp Lys Gln Ile Arg Tyr Ile
            20                  25                  30
CTC GAC GGC ATC TCA GCC TTA AGA AAG GAG ACA TGT AAC AAG AGT AAC 144
Leu Asp Gly Ile Ser Ala Leu Arg Lys Glu Thr Cys Asn Lys Ser Asn
        35                  40                  45
ATG TGT GAA AGC AGC AAA GAG GCA CTG GCA GAA AAC AAC CTG AAC CTT 192
Met Cys Glu Ser Ser Lys Glu Ala Leu Ala Glu Asn Asn Leu Asn Leu
    50                  55                  60
CCA AAG ATG GCT GAA AAA GAT GGA TGC TTC CAA TCT GGA TTC AAT GAG 240
Pro Lys Met Ala Glu Lys Asp Gly Cys Phe Gln Ser Gly Phe Asn Glu
65                  70                  75                  80
GAG ACT TGC CTG GTG AAA ATC ATC ACT GGT CTT TTG GAG TTT GAG GTA 288
Glu Thr Cys Leu Val Lys Ile Ile Thr Gly Leu Leu Glu Phe Glu Val
                85                  90                  95
TAC CTA GAG TAC CTC CAG AAC AGA TTT GAG AGT AGT GAG GAA CAA GCC 336
Tyr Leu Glu Tyr Leu Gln Asn Arg Phe Glu Ser Ser Glu Glu Gln Ala
            100                 105                 110
AGA GCT GTC CAG ATG AGT ACA AAA GTC CTG ATC CAG TTC CTG CAG AAA 384
Arg Ala Val Gln Met Ser Thr Lys Val Leu Ile Gln Phe Leu Gln Lys
        115                 120                 125
AAG GCA AAG AAT CTA GAT GCA ATA ACC ACC CCT GAC CCA ACC ACA AAT 432
Lys Ala Lys Asn Leu Asp Ala Ile Thr Thr Pro Asp Pro Thr Thr Asn
    130                 135                 140
GCC AGC CTG CTG ACG AAG CTG CAG GCA CAG AAC CAG TGG CTG CAG GAC 480
Ala Ser Leu Leu Thr Lys Leu Gln Ala Gln Asn Gln Trp Leu Gln Asp
145                 150                 155                 160
ATG ACA ACT CAT CTC ATT CTG AGA TCT TTT AAG GAG TTC CTG CAG TCC 528
Met Thr Thr His Leu Ile Leu Arg Ser Phe Lys Glu Phe Leu Gln Ser
                165                 170                 175
AGC CTG AGG GCT CTT CGG CAA ATG TAG 555
Ser Leu Arg Ala Leu Arg Gln Met
            180

(2)     INFORMATION FOR SEQ ID NO: 2
(i)     SEQUENCE CHARACTERISTICS
(A) LENGTH: 70 base pairs
(B) TYPE: nucleotide
(C) STRANDEDNESS: single stranded
(D) TOPOLOGY: linear
(ii)    MOLECULE TYPE: synthetic DNA
(iii)   HYPOTHETICAL: no
(iv)    ANTISENSE: no
(v)     FRAGMENT TYPE: internal
(vii)   IMMEDIATE SOURCE
(A) SYNTHESIS: oligonucleotide synthetiser
(ix)    FURTHER CHARACTERISTICS
(A) NAME: IL-6 31D35 PCR
(C) IDENTIFICATION METHOD: polyacrylamide gel
(xi)    SEQUENCE DESCRIPTION: SEQ ID NO: 2
CGCACGAGCT CAGAACGAAT TGACAAACAA ATTCGGKACA TCCTCGACYD  TATCTCAGCC  60
TTAAGAAAGG                                                          70
(3) INFORMATION FOR SEQ ID NO:3
(i) SEQUENCE CHARACTERISTICS
(A) LENGTH:          72 nucleotides
(B) TYPE:            nucleotidic
(C) STRANDEDNESS:    single
(D) TOPOLOGY:        linear
(ii)  MOLECULE TYPE:     synthetic DNA
(iii) HYPOTHETICAL:      no
(iv)  ANTISENSE:         yes
(v)   FRAGMENT TYPE:     internal
(vii) IMMEDIATE SOURCE:
(A) SYNTHESIS:     oligonucleotidic synthetizer
(ix) FURTHER CHARACTERISTICS
(A) NAME: IL-6 118RCLF/121VD
(C) IDENTIFICATION METHOD:   polyacrylamide gel
(xi) SEQUENCE DESCRIPTION: SEQ ID NO:3
CGCATCTAGA TTCTTTGCCT TTTTCTGCAG GAACTGGATC AGGKCTTTTG TGYZCATCTG  60
CACAGCTCTG GC                                                      72
INFORMATION FOR SEQ ID NO:4

(i) SEQUENCE CHARACTERISTICS
(A) LENGTH: 62 nucleotides
(B) TYPE: nucleotide
(C) STRANDEDNESS: single stranded
(D)TOPOLOGY: linear
(ii) MOLECULE TYPE: synthetic DNA
(iii) HYPOTHETICAL: no
(iv) ANTISENSE: yes
(v) FRAGMENT TYPE: carboxi-terminal
(vii)IMMEDIATE SOURCE
(A) SYNTHESIS: oligonucleotide synthesiser
(ix) FURTHER CHARACTERISTICS
(A) NAME : IL-6 QSLQ (AS)
(C) IDENTIFICATION METHOD: polyacrylamide gel
(xi) SEQUENCE DESCRIPTION: SEQ ID NO:4
CCGGGCGGCC GCCCTACATM NNCCGMNNAG CCCTCAGMNN GGAMNNCAGG AACTCCTTAA 60
AG 62

INFORMATION FOR SEQ ID NO: 5
(i) SEQUENCE CHARACTERISTICS
(A) LENGTH: 24 nucleotides
(B) TYPE: nucleotide
(C)STRANDEDNESS: single stranded
(D)TOPOLOGY: linear
(ii) MOLECULE TYPE: synthetic DNA
(iii) HYPOTHETICAL: no
(iv) ANTISENSE: no
(v) FRAGMENT TYPE: internal
(vii) IMMEDIATE SOURCE
(A) SYNTHESIS: oligonucleotide synthesiser
(ix) FURTHER CHARACTERISTICS
(A) NAME : IL-6 QSLQ pr. Bam
(C) IDENTIFICATION METHOD: polyacrylamide gel
(xi) SEQUENCE DESCRIPTION: SEQ ID NO: 5
CGCGGATCCT TTAAGGAGTT CCTG 24
INFORMATION FOR SEQ ID NO: 6
(i) SEQUENCE CHARACTERISTICS

(A) LENGTH: 73 nucleotides
(B) TYPE: nucleotide
(C) STRANDEDNESS: single stranded
(D) TOPOLOGY: linear
(ii) MOLECULE TYPE: synthetic DNA
(iii) HYPOTHETICAL: no
(iv) ANTISENSE: no
(v) FRAGMENT TYPE: carboxi-terminal
(vii) IMMEDIATE SOURCE
(A) SYNTHESIS: oligonucleotide synthesiser
(ix) FURTHER CHARACTERISTICS
(A) NAME : 175I/167R/183A (S)
(C) IDENTIFICATION METHOD: polyacrylamide gel
(xi) SEQUENCE DESCRIPTION: SEQ ID NO: 6
GCCTGAGATC TTTTAAGGAG TTCCTGATCC GTAGCCTGAG GGCTCTTCGG GCTATGTAGG 60
GCGGCCGCAT GGC 73
INFORMATION FOR SEQ ID NO: 7
(i) SEQUENCE CHARACTERISTICS
(A) LENGTH: 73 nucleotides
(B) TYPE: nucleotide
(C)STRANDEDNESS: single stranded
(D)TOPOLOGY: linear
(ii) MOLECULE TYPE: synthetic DNA
(iii) HYPOTHETICAL: no
(iv) ANTISENSE: yes
(v) FRAGMENT TYPE: internal
(vii) IMMEDIATE SOURCE
(A) SYNTHESIS: oligonucleotide synthesiser
(ix) FURTHER CHARACTERISTICS
(A) NAME : 175I/167R/183A (AS)
(C) IDENTIFICATION METHOD: polyacrylamide gel
(xi) SEQUENCE DESCRIPTION: SEQ ID NO: 7
GCCATGCGGC CGCCCTACAT AGCCCGAAGA GCCCTCAGGC TACGGATCAG GAACTCCTTA 60
AAAGATCTCA GGC 73

## Claims

1. A methodology for selecting superagonists, antagonists and superantagonists of interleukin 6 using the membrane molecule gp 130 to activate the mechanisms regulating cell physiology, comprising the following operations:
- comparing the amino acid sequences of the growth hormone with the sequences of interleukin 6;
- comparing the amino acid sequences of the growth hormone receptor with those of the two receptors of interleukin 6, that is with the interleukin 6-specific receptor and gp 130;
- on the basis of the above comparisons, formulating a three-dimensional model of the receptor complex based on the
functional similarity between sequences on the growth hormone receptor and the two interleukin 6 receptors in question;
- identifying the residues of the wild type interleukin 6 that form a part of the site of interaction with the specific receptor and the site of interaction with gp 130, respectively;
introduction of mutations into the interaction sites of interleukin 6; and
- evaluation of the biological activity of the mutants.

2. The methodology for selecting superagonists of interleukin 6 according to claim 1, further comprising the following additional operations:
- production of a series of phage libraries containing mutations of the following wild type residues of interleukin 6 present in the form of a fusion product with filamentous phage proteins
Glu 42, Glu 51, Ser 52, Ser 53, Lys 54, Glu 55, Asn 63, Lys 66, Met 67, Ala 68, Glu 69, Lys 70, Asp 71, Phe 170, Gln 175, Ser 176, Ser 177, Leu 181, Gln 183;
- generation of a phage library, each phage having a mutant interleukin 6 sequence;
- selection, from the mixed population of phages expressing interleukin 6 mutants, of that or those with an affinity for the specific receptor greater than that of wild type interleukin; and
- identification of the best amino acid sequence or sequences binding the receptor by sequencing of the DNA extracted from the selected phage particles.

3. The methodology for selecting superagonists of interleukin 6 according to claim 2, in which a series of phage libraries are produced, containing mutants of said wild type residue of interleukin 6 present as a product of fusion with protein pIII of M13.

4. The methodology for selecting antagonists of interleukin 6 according to claim 1 further comprising the following additional operations:
- mutation of the residues identified in claim 1, to form part of the site of interaction with gp 130 (Arg 30, Tyr 31, Gly 35, Ser 37, Ala 38, Ser 118, Lys 120, Val 121, Gln 124, Phe 125, Gln 127, Lys 128 and Lys 129), using conventional molecular biology techniques;
- evaluation of biological activity and affinity with the specific interleukin 6 receptor of the mutants produced as above, in order to identify variants of interleukin 6 whose affinity to the specific receptor is intact and that show reduction or loss of the biological activity; and
- evaluation of the above variants of interleukin 6 as antagonists for the biological activity of wild type interleukin 6.

5. The methodology for selecting superantagonists of interleukin 6 according to claim 1 by combination of the variations of amino acid sequences responsible for antagonist activity, which are indicated in claim 4, with amino acid variations responsible for an increased affinity of the specific receptor for interleukin 6.

6. A methodology for selecting superantagonists of interleukin 6 according to claim 5, in which the mutagenesis of the residues identified as above is performed using a molecular biology technique chosen from the group comprising Polymerase Chain Reaction, Primer Extension, Oligonucleotide Directed Mutagenesis, and combinations thereof.

## Patentansprüche

1. Verfahren zum Auswählen von Superagonisten, Antagonisten und Superantagonisten von Interleukin 6, wobei das Membranmolekül gp 130-verwendet wird, um die Mechanismen zu aktivieren, welche die Zellphysiologie regulieren, umfassend die folgenden Schritte:
- Vergleichen der Aminosäuresequenzen des Wachstumshormons mit den Sequenzen von Interleukin 6;
- Vergleichen der Aminosäuresequenzen des Wachstumshormonrezeptors mit denen der zwei Rezeptoren von Interleukin 6, d. h. mit dem Interleukin 6-spezifischen Rezeptor und gp 130;
- Formulieren eines drei-dimensionalen Models des Rezeptorkomplexes auf der Grundlage der obigen Vergleiche, basierend auf der funktionalen Ähnlichkeit zwischen Sequenzen des Wachstumshormonrezeptors und denen der vorliegenden zwei Interleukin 6 Rezeptoren;
- Identifizieren der Aminosäurereste des Wildtyp-Interleukin 6, die einen Bestandteil der Interaktionsstelle mit dem spezifischen Rezeptor, beziehungsweise der Interaktionsstelle mit gp 130 bilden,
- Einfüren von Mutationen in die Interaktionsstellen von Interleukin 6, und Auswerten der biologischen Aktivität der Mutanten.

2. Verfahren zum Auswählen von Superagonisten von Interleukin 6 gemäß Anspruch 1, weiterhin umfassend die folgenden zusätzlichen Schritte:
- Herstellen einer Serie von Phagenbibliotheken, die Mutationen der-folgenden Wildtypaminosäurereste von Interleukin 6 enthalten, welche in Form eines Fusionsproduktes mit Proteinen filamentöser Phagen vorliegen
Glu 42, Glu51, Ser 52, Ser 53, Lys 54, Glu 55, Asn 63, Lys 66, Met 67, Ala 68, Glu 69, Lys 70, Asp 71, Phe 170, Gln 175, Ser 176, Ser 177, Leu 181, Gln 183;
- Herstellen einer Phagenbibliothek, wobei jeder Phage eine Mutanten-Interleukin 6 Sequenz aufweist;
- Auswählen aus der gemischten Population von Phagen, die Interleukin 6 Mutanten exprimieren, von solchen, die eine Affinität für die spezifischen Rezeptoren aufweisen, die größer ist als die von Wildtyp-Interleukin; und
- Identifizieren der besten Aminosäuresequenz oder -sequenzen, die den Rezeptor binden, durch Sequenzleren der extrahierten DNA aus den ausgewählten Phagenpartikeln.

3. Verfahren zum Auswählen von Superagonisten von Interleukin 6 gemäß Anspruch 2, in welchem eine Serie von Phagenbibliotheken hergestellt wird, die Mutanten des genannten Wildtyp-Aminosäurerests von Interleukin 6 enthalten, welche als ein Fusionsprodukt mit Protein pIII von M13 vorliegen.

4. Verfahren zum Auswählen von Antagonisten von Interleukin 6 gemäß Anspruch 1 weiterhin umfassend die folgenden zusätzliche Schritte:
- Mutieren der in Anspruch 1 identifizierten Aminosäurereste, die einen Teil der Interaktionsstelle mit gp 130 bilden (Arg 30, Tyr 31, Gly 35, Ser 37, Ala 38, Ser 118, Lys 120, Val 121, Gln 124, Phe 125, Gln 127, Lys 128 und Lys 129), unter Anwendung von üblichen molekularbiologischen Techniken;
- Auswerten der biologischen Aktivität und der Affinität zu dem spezifischen Interleukin 6 Rezeptor der wie oben erzeugten Mutanten, um Varianten von Interleukin 6 zu identifizieren, deren Affinität zu dem spezifischen Rezeptor intakt ist und die eine Reduzierung oder einen Verlust der biologischen Aktivität aufweisen, und
- Auswerten der obigen Varianten von Interleukin 6 als Antagonisten für die biologische Aktivität von Wildtyp-Interleukin 6.

5. Verfahren zum Auswählen von Superantagonisten von Interleukin 6 gemäß Anspruch 1 durch Kombination der Varianten von Aminosäuresequenzen, die für die Antagonistaktivität verantwortlich sind, welche in Anspruch 4 angeführt sind, mit Aminosäure-Varianten, die für eine erhöhte Affinität des spezifischen Rezeptors für Interleukin 6 verantwortlich sind.

6. Verfahren zum-Auswählen von Superantagonisten von Interleukin 6 gemäß Anspruch 5, wobei die Mutagenese der wie oben identifizierten Aminosäurereste unter Verwendung einer molekularbiologischen Technik durchgeführt wird, ausgewählt aus der Gruppe umfassend Polymerase-Kettenreaktion, Primer-Extension, gerichtete Mutagenese durch Oligonukleotide und Kombinationen davon.

## Revendications

1. Procédé de sélection de superagonistes, d'antagonistes et de superantagonistes de l'interleukine 6 utilisant la molécule membranaire gp 130 pour activer les mécanismes de régulation de la physiologie des cellules, comprenant les opérations suivantes :
- la comparaison des séquences d'aminoacides de l'hormone de croissance avec les séquences de l'interleukine 6 ;
- la comparaison des séquences d'aminoacides du récepteur de l'hormone de croissance avec celles des deux récepteurs de l'interleukine 6, à savoir celles du récepteur spécifique de l'interleukine 6 et de la gp 130 ;
- d'après les comparaisons précédentes, la formulation d'un modèle à trois dimensions du complexe récepteur se fondant sur la similitude fonctionnelle entre les séquences du récepteur de l'hormone de croissance et celles des deux récepteurs de l'interleukine 6 en question ;
- l'identification des résidus de l'interleukine 6 de type sauvage qui forment respectivement une partie du site d'interaction avec le récepteur spécifique et du site d'interaction avec la gp 130 ;
- l'introduction de mutations dans les sites d'interaction de l'interleukine 6 ; et
- l'évaluation de l'activité biologique des mutants.

2. Procédé de sélection de superagonistes de l'interleukine 6 selon la revendication 1, comprenant en outre les opérations supplémentaires suivantes :
- la production d'une série de banques de phages contenant des mutations des résidus de type sauvage suivants de l'interleukine 6, présente sous la forme d'un produit de fusion avec des protéines de phages filamenteux : Glu 42, Glu 51, Ser 52, Ser 53, Lys 54, Glu 55, Asn 63, Lys 66, Met 67, Ala 68, Glu 69, Lys 70, Asp 71, Phe 170, Gln 175, Ser 176, Ser 177, Leu 181, Gln 183 ;
- la production d'une banque de phages dont chaque phage a une séquence d'interleukine 6 mutante ;
- la sélection, à partir de la population mixte de phages exprimant des mutants de l'interleukine 6, de celui ou de ceux ayant une affinité pour le récepteur spécifique plus grande que celle de l'interleukine 6 de type sauvage ; et
- l'identification de la meilleure ou des meilleures séquences d'aminoacides se liant au récepteur par séquençage de l'ADN extrait des particules de phages sélectionnées.

3. Procédé de sélection de superagonistes de l'interleukine 6 selon la revendication 2, dans laquelle on produit une série de banques de phages contenant des mutants dudit résidu de type sauvage d'interleukine 6 présents sous forme de produits de fusion avec la protéine pIII de M13.

4. Procédé de sélection d'antagonistes de l'interleukine 6 selon la revendication 1, comprenant en outre les opérations supplémentaires suivantes :
- la mutation des résidus identifiés dans la revendication 1 comme formant une partie du site d'interaction avec la gp 130 (Arg 30, Tyr 31, Gly 35, Ser 37, Ala 38, Ser 118, Lys 120, Val 121, Gln 124, Phe 125, Gln 127, Lys 128 et Lys 129), à l'aide de techniques classiques de biologie moléculaire ;
- l'évaluation de l'activité biologique et de l'affinité pour le récepteur spécifique de l'interleukine 6 des mutants produits ci-dessus, pour identifier des variantes de l'interleukine 6 dont l'affinité pour le récepteur spécifique est intacte et qui présentent une réduction ou une perte de l'activité biologique;
- l'évaluation des variantes de l'interleukine 6 ci-dessus comme antagonistes de l'activité biologique de l'interleukine 6 de type sauvage.

5. Procédé de sélection de superantagonistes de l'interleukine 6 selon la revendication 1 par combinaison des variations de séquences d'aminoacides responsables de l'activité antagoniste indiquées dans la revendication 4 avec des variations d'aminoacides responsables d'une augmentation de l'affinité du récepteur spécifique pour l'interleukine 6.

6. Procédé de sélection de superantagonistes de l'interleukine 6 selon la revendication 5, dans laquelle la mutagenèse des résidus identifiés de la manière décrite ci-dessus est effectuée à l'aide d'une technique de biologie moléculaire choisie dans le groupe comprenant l'amplification en chaîne par polymérase, l'allongement d'amorces, la mutagenèse dirigée avec un oligonucléotide, et leurs combinaisons.
